# EUROPEAN PATENT APPLICATION

(11) **EP 1 518 934 A1**
(43) Date of publication of application: **30.03.2005**
(21) Application number: 03292392.2
(22) Date of filing: 29.09.2003
(51) Int. Cl.: C12Q 1/68

(54) **Identification of polymorphisms in the EPCR gene associated with thrombotic risk**

(71) Applicant: INSTITUT NATIONAL DE LA SANTE ET DE LA RECHERCHE MEDICALE (INSERM), 75013 Paris (FR)
(72) Inventor: Aiach, Martine, 92310 Sevres (FR); Saposnik, Béatrice, 75012 Paris (FR); Emmerich, Joseph, 75001 Paris (FR); Gandrille, Sophie, 75015 Paris (FR); Gaussem, Pascale, 94240 L'Hay Les Roses (FR)
(74) Representative: Bernasconi, Jean

(57) **Abstract**

The invention relates to an *in vitro* method for determining the risk of developing thrombosis in a subject, which method involves identifying a particular haplotype of the EPCR gene.

SEQUENCE LISTING

<110> INSERM

<120> Identification of polymorphisms in the EPCR gene
      associated with thrombotic risk

<130> BET 03P0959

<140>
<141>

<160> 18

<170> PatentIn Ver. 2.1

<210> 1
<211> 8167
<212> DNA
<213> Homo sapiens

<400> 1
aaatgaaata tttcaggctg tgcacagtgg ctcaggcttg taatcccagc atgttgggag 60
gctgaagtgg gcggatcacc tgaggtcagg agtttgagac caacctggcc aacatggtga 120
aatcccatct ctactaaaaa tacaaaaatt agccaggtgt ggtggcaggt gactgtaatc 180
ccagctactt gggaggctga ggcaggagaa tcgcttgaat ctgggaggtg gaggttgcag 240
tgagccgaga tcacgccact gcatacagca agactccatc tcaaaaaaaa gaaaaaaaaa 300
aagaaaaaag aaatgtttca taatttttaa taaaaggcaa gacaatataa attggtagtt 360
atttaagtca ttctactttt cctgaggccc agtgcaggaa aacaaagttc ctatccttgt 420
tccaactaga ccattttgat aagctgcaaa aagaaaagac tttgatgcta tttcttagcc 480
agtttgcaac agctgagagg tgagcatgga agctcttgca tatattcagt tcagagaatg 540
ggtgcttagt ttatgtccag agtttgtccc agatttcact atgacgtcag ctctccgggg 600
agaagtatat aaaataaaaa gttaaaatcc ctctcagtcc tttacccaat cctattcccc 660
agaggtaatc tctattgaca gtacccctcc agatattttc cctatgtata tacaaataca 720
cagatacaca ctgaaagtta attttggcca ggtgcagtgg ctcctgccta taccagagga 780
ttgcttgagt gcaggagttc aagaccagcc tgggcaacat agcgagacca catctctagt 840
aaaaataaaa aaaaaatagc taggcgtggt ggcacagtgg cacgtacctt tagtctcagc 900
tactcgggtg gttgaggtgg gagaatcact tgagcccggg aggtcaagcc tacaattagc 960
tgtgattgct tcactgcact atagcctggg caacagagct agaccctgtc tcaaaaaaat 1020
aataataaat tttatatata tatatgagga tgaaattaca tatgtattat ttgaacagaa 1080
gtgaaatctt ttcttttttt ttttcagaca gaatcttgcc gcatgaccca ggctagaatg 1140
cagtggtgtg atctcggccc tctgcaacct ccacctccca ggttcaagcg attctcatgc 1200
ctcggtctcc caagtagctg ggattacagg catgcaccac catgcccagc taatttttgt 1260
atttttcgta gagacgttcg ccatattggc caggctggtc tcaaactcct ggcctcaagt 1320
gatctgccca cctcggcctc ccaaagtgcc agcagcatgc tcggaggagt gactttaaag 1380
cttttctact tgcttcctag agtaagggac gcattttaca ctgctatcca aaactcatca 1440
tagaaacata cacacacaaa accaaagcac acatatacaa ctgagcaaat atttcatgac 1500
ataacacttt ctcttactaa gggtgacgcg ctgaaatttt gtattctgtc ctatttcatt 1560
ttttaaaaat ggtaaccatg acctgctaaa ttgatttcat tgtccactaa taaattatga 1620
cctcagtttc aaaaagattg ctttaggtaa ccaatcatct tctgagattt atacagattg 1680
ctcataattc tctcctattt tttaaaaaca tgctgcagtg aactgcttta cactcatttt 1740
atgactactt ctgagaccaa gatcccggat tatgtaattg ttatttactt aaaattctgg 1800
taaaatgtag ccattatact ggaaaactaa attttaatct tggatctgtc accaccatga 1860
tatataaact ttgggcaagt ccctgcacct ctctggacct caatctcccc atcagcaacc 1920
tgctgatcct actcccagga gtgtgctcta agttgaaagt agatgcccca ccccctgagt 1980
cagcgccggc aggacttctc accaagccct tctccccctt ttccgctccc tgttcctggt 2040
tcctaggaag cagcccaagg agaagggaaa aggcaggtct gggcaggagg gagcaatgaa 2100
gggcggggca gagggagggc aggagggagg ccggccccct agtaggaaat gagacacagt 2160
agaaataaca ctttataagc ctcttcctcc tcccatctcc tggcctcctt ccatcctcct 2220
ctgcccagac tccgcccctc ccagacggtc ctcacttctc ttttccctag actgcagcca 2280
gcggagcccg cagccggccc gagccaggaa cccaggtccg gagcctcaac ttcaggatgt 2340

tgacaacatt gctgccgata ctgctgctgt ctggctgggc cttttgtagc caagacgcct 2400
cagatggtga gtcgggggca catctcctgc ctcaggatgg ttctggagaa tctcagtcta 2460
tctgggcaca tggcaagacc acaggagagc ttatctcaca gcatctgtgt ctgcagctgg 2520
ctagatctct ctacagggca ggcagagtct tggggactgg ttcgtgtccc aaagccaagg 2580
tgagttagta catttaagcc cctgaaaagg gggagatgaa agaggctagg ggaaacagga 2640
tgactggaaa catgagaaag aaaccagcag agagggtagg agaatcagcc ccagggagag 2700
gggagaaagg ggaactgagg gtgatggtag ataggggtac atctagggga gacgggaaga 2760
ggctcagaag agaagagaaa tggagggaat gggaagaccc tgggaaaact gatggaagaa 2820
gtgggggaag agtggggcag agagaggtta ggggaggcta gggaaaatgg aaggagactg 2880
gtcgcagctg gtggaactgg ggagaaagag atgctgtgcc taatagaact tatgggcgat 2940
caggctactg aagtggccct gtttaagcag aaaagggagt tattaccctc cattataatt 3000
gcacaggggc ctcctttccc ctctctcaca atccccgtaa cttcagtctc cccctcagag 3060
aggcagcaaa taataaccag tattcaatga gtgctcacta tggttaatac atgtattgac 3120
ccatttaact tgcacaaacc cctaaaggtg ggtaatatta ttactatctc cattttatga 3180
ggaggaaact gggtcacaga gtagttaagg accatgtcta gggttatcca taaatatact 3240
tattcacatc tgcagataca aagcacaact tctcaaatgc aaacacagac aggacccact 3300
cacacacaca gatttacaac cccggactca tccaaatgtg ctctgggcat caactctgtg 3360
ccagcctctt ttctgggtgt aggaagcaga gattaccaag catggttcca tagcctagag 3420
gagtccagtg tggcctgtgt gtgtttggag acagccaggt agtatcccgt gagatacaca 3480
ctaatatatg gtggtctggg atcactgaaa cagacacact gtgtctcgtg gggcatcaga 3540
aaaaaatttc caagaagagg gcaactgagc tgggtctttt tttctttgct tttctttctt 3600
ttttcttttt tttttttttt tttttttttg agatggagtc ttgtgctgtc acccaggctg 3660
gaatgcagtg gcacaatttc agctaactgt aacctccaac tcccaggttc aggcgattct 3720
cctgcctcag cctcctgagt agctgggact acaggcatgt accaccacgc ctggctaata 3780
tttgtacttt tagtacagat ggggtttcgc catgttggcc aggctggtct tgaatccctg 3840
acctcaagtg atccgcccgc ctcggcctcc caaagtgctg ggattacagg catgagccac 3900
cgcgcccagt ctctgagctg ggtcttaaat catgaataaa cttcgccagg cagaaaaagg 3960
gaggcagagc aatcctgaca tgctattcat gtgtcagcca aaggcagcat gaggaatccc 4020
aactagtttg atatataagc agcgggaagc ggccagaaaa ggcagcaggg gccaggtctc 4080
tagcagcctt gaatgccagg ctaaagactc tggacttgat cctgtgggga ggcagtgtag 4140
cagaatggct gagtgctgga cttgactgcc tacgtgcaaa ccttggctct gctacactat 4200
ctctgtctca gtttcgcatg tagactgggg ttaataatag tagctattgc attaagccac 4260
tggggaaagg cacaaagata ataatgtatg taaagcccat tgcccaggtt ataataagca 4320
ctgaatcgac attggctatg attatttttg attaatgaag gggagggggt tatggcactg 4380
gaagatttta agtaggaaaa ggacatgatc tcatccctgg gtcaggtgga ggtcggaata 4440
gagaacgggg agatgaagta gaaagttact accccagtct agatgagacg gatgaatcct 4500
gaatcagggc agtggaagag gagatggaga acaggcgatg gaattggaat tttattcagg 4560
tcaggatttg ttaaccattt gttccgttgg ttaacaggaa acggggggag ggagagccga 4620
gggtgaaaaa ggaggcagaa aggagtgtct cttccactgc aggcctcagt ttcctcatct 4680
gtaaaacgga gataataatc cctgtcctgt cctcctggca gagttactgt cagcgtcaaa 4740
cgggagaagc ggtgggaggg cacattatag tttatgaagg gtcgagaagg cgggcggcca 4800
gcctcgaggt agggggttat tatcttccgc tgcccgccgc cccctcccac gccggcccag 4860
gctgaagttg actctgcccg caggcctcca aagacttcat atgctccaga tctcctactt 4920
ccgcgacccc tatcacgtgt ggtaccaggg caacgcgtcg ctggggggac acctaacgca 4980
cgtgctggaa ggcccagaca ccaacaccac gatcattcag ctgcagccct tgcaggagcc 5040
cgagagctgg gcgcgcacgc agagtggcct gcagtcctac ctgctccagt tccacggcct 5100
cgtgcgcctg gtgcaccagg agcggacctt ggcctgtgag taggcgcgca gcgggggcgg 5160
ggtctgggcg gggctagtgg gggcggggcc tggcgggtgg gggcggggcc tggcggatgg 5220
aggcgggctg gggcttgcag ggacccggca gccactggag ctcggtggcg cctgggcctt 5280
tgaagattgc tgggtggggg ctggagagag gcagttgtcc ccgctaagaa agccccgact 5340
cgggcggtcg tcctgctggc ataacctctt gggatagacc ctgttggaag gccctgacac 5400
cgtgacgtcg aaggtcccca gaaaactcct cacccctcgc ctcacagtcc tccaactcct 5460
tttcttcata gatctccgtc cttcccttcc cacagccccc agcacttcac cccccaccct 5520
ccagccactt ctcatacaag ctgatgactt cgctcttagc tccactcatg acccgaactc 5580
ttcccccaaa gaccccaagt tcttctctca aagccccact ccttccccgt cacaacccta 5640
actccttctt ctcaaagacc ccaatttctt ttctcaaagc accaagcacc actccgtccc 5700
ccttccccca ccatcatggc ctttaattcc tttctctcct agtcccccac cccaccccct 5760
tttttttttt tttttttttt tttttttgag acggagtctt gctctgtcgt ccaggctgga 5820
gtgcagtggc gcgatctcgg ctcactgcaa cttccgcctc ccgggttcaa gcgattctcc 5880
tgcctcagcc tcccaagcag ctgggactac aggcacccgc caccacgccc ggctaatttt 5940
ttgtattttt agtagagacg gggtttcgcc atgttggcca ggctggtctc gaactcctga 6000

cctcaggcga tccacaagcc tggcctccca aagtgctggg attacaggcg tgagctgccg 6060
cccctgcccc agcctcaccc cctgtttttt ttttctatta cagttgaaca aggcctgaca 6120
attccctttt ttcatcacag tccctggccc cttctttctt agcctctaac aggctaaccc 6180
caaacccctc ctcacagccc caggcccttc tccccatagt tccctgacct agactcccct 6240
ctcctcacag cactgactct tgccttctca tgttcttttc cccttggtgg gcctcgcccc 6300
acacctggca ccctctctgc acagtcccct gatcctgact gtctatccac agttcctctg 6360
accatccgct gcttcctggg ctgtgagctg cctcccgagg gctctagagc ccatgtcttc 6420
ttcgaagtgg ctgtgaatgg gagctccttt gtgagtttcc ggccggagag agccttgtgg 6480
caggcagaca cccaggtcac ctccggagtg gtcaccttca ccctgcagca gctcaatgcc 6540
tacaaccgca ctcggtatga actgcgggaa ttcctggagg acacctgtgt gcagtatgtg 6600
cagaaacata tttccgcgga aaacacgaaa ggtatgatgg gacggggccc aggcctgcaa 6660
gctggggaga gggcgggttc cagacaaatg gatggacctg aaggatggat gcctagagca 6720
acaagaggcc cacagctggg ggtttgggac agaacacacg cagcttcagt cagttggtaa 6780
acgggtccct ttcctctggg gcagaaacgc tttggggttt gactcaaatc atggactcct 6840
tgggggccta ttcttcgggc taactctttg catgttctgc agggagccaa acaagccgct 6900
cctacacttc gctggtcctg ggcgtcctgg tgggcagttt catcattgct ggtgtggctg 6960
taggcatctt cctgtgcaca ggtggacggc gatgttaatt actctccagc cccgtcagaa 7020
ggggctggat tgatggaggc tggcaaggga aagtttcagc tcactgtgaa gccagactcc 7080
ccaactgaaa caccagaagg tttggagtga cagctccttt cttctcccac atctgcccac 7140
tgaagatttg agggagggga gatggagagg agaggtggac aaagtacttg gtttgctaag 7200
aacctaagaa cgtgtatgct ttgctgaatt agtctgataa gtgaatgttt atctatcttt 7260
gtggaaaaca gataatggag ttggggcagg aagcctatgg cccatcctcc aaagacagac 7320
agaatcacct gaggcgttca aaagatataa ccaaataaac aagtcatcca caatcaaaat 7380
acaacattca atacttccag gtgtgtcaga cttgggatgg gacgctgata taatagggta 7440
gaaagaagta acacgaagaa gtggtggaaa tgtaaaatcc aagtcatatg gcagtgatca 7500
attattaatc aattaataat attaataaat ttcttatatt taaggcattg ttatctcctc 7560
cactttgcaa aatttctgga aaagtaacct atacccattt cttctgcttc cttatttctc 7620
actcattctt tttttttttt tttttttttt ttgagacaga gtcttgctct gttgcctagg 7680
ctggagtgca atggtgtgat ctcagctcac tgcaacctct gcctcccggt tcaagcaatt 7740
ctcctgcctc agcctcccaa gcagctggga ttacagatgc atgccaccac acccagctaa 7800
tttttgtatt tttagtagag atggggtttc accacgttgg ccatcctgac ctcgtgatcc 7860
gcctacctcg gcctccccaa gtgctgggat tagacgtgag ccactgcgcc tggtcttctc 7920
actcattctt agacccagtg caatctgact tctctataaa ctactctaag atcaccagta 7980
acctctaatt gtcaaaccgt caccctacat ggtatctgca aatttgcgga ctagaactct 8040
ctttttgcct taacttctga gataccatac ttcaattttt aaaactgttc tgtctacttt 8100
ttttcaatcc ctttgactat gtcatcttac acgattcacc ctggaaatgc tggcttcctt 8160
agaattc 8167

<210> 2
<211> 8167
<212> DNA
<213> Homo sapiens

<400> 2
aaatgaaata tttcaggctg tgcacagtgg ctcaggcttg taatcccagc atgttgggag 60
gctgaagtgg gcggatcacc tgaggtcagg agtttgagac caacctggcc aacatggtga 120
aatcccatct ctactaaaaa tacaaaaatt agccaggtgt ggtggcaggt gactgtaatc 180
ccagctactt gggaggctga ggcaggagaa tcgcttgaat ctgggaggtg gaggttgcag 240
tgagccgaga tcacgccact gcatacagca agactccatc tcaaaaaaaa gaaaaaaaaa 300
aagaaaaaag aaatgtttca taatttttaa taaaaggcaa gacaatataa attggtagtt 360
atttaagtca ttctactttt cctgaggccc agtgcaggaa aacaaagttc ctatccttgt 420
tccaactaga ccattttgat aagctgcaaa aagaaaagac tttgatgcta tttcttagcc 480
agtttgcaac agctgagagg tgagcatgga agctcttgca tatattcagt tcagagaatg 540
ggtgcttagt ttatgtccag agtttgtccc agatttcact atgacgtcag ctctccgggg 600
agaagtatat aaaataaaaa gttaaaatcc ctctcagtcc tttacccaat cctattcccc 660
agaggtaatc tctattgaca gtacccctcc agatattttc cctatgtata tacaaataca 720
cagatacaca ctgaaagtta atttggccaa ggtgcagtgg ctcctgccta taccagagga 780
ttgcttgagt gcaggagttc aagaccagcc tgggcaacat agcgagacca catctctagt 840
aaaaataaaa aaaaaatagc taggcgtggt ggcacagtgg cacgtacctt tagtctcagc 900
tactcgggtg gttgaggtgg gagaatcact tgagcccggg aggtcaagcc tacaattagc 960

tgtgattgct tcactgcact atagcctggg caacagagct agaccctgtc tcaaaaaaat 1020
aataataaat tttatatata tatatgagga tgaaattaca tatgtattat ttgaacagaa 1080
gtgaaatctt ttcttttttt ttttcagaca gaatcttgcc gcatgaccca ggctagaatg 1140
cagtggtgtg atctcggccc tctgcaacct ccacctccca ggttcaagcg attctcatgc 1200
ctcggtctcc caagtagctg ggattacagg catgcaccac catgcccagc taatttttgt 1260
atttttcgta gagacgttcg ccatattggc caggctggtc tcaaactcct ggcctcaagt 1320
gatctgccca cctcggcctc ccaaagtgcc agcagcatgc tcggaggagt gactttaaag 1380
cttttctact tgcttcctag agtaagggac gcattttaca ctgctatcca aaactcatca 1440
tagaaacata cacacacaaa accaaagcac acatatacaa ctgagcaaat atttcatgac 1500
ataacacttt ctcttactaa gggtgacgcg ctgaaatttt gtattctgtc ctatttcatt 1560
ttttaaaaat ggtaaccatg acctgctaaa ttgatttcat tgtccactaa taaattatga 1620
cctcagtttc aaaaagattg ctttaggtaa gcaatcatct tctgagattt atacagattg 1680
ctcataattc tctcctattt tttaaaaaca tgctgcagtg aactgcttta cactcatttt 1740
atgactactt ctgagaccaa gatcccggat tatgtaattg ttatttactt aaaattctgg 1800
taaaatgtag ccattatact ggaaaactaa attttaatct tggatctgtc accaccatga 1860
tatataaact ttgggcaagt ccctgcacct ctctggacct caatctcccc atcagcaacc 1920
tgctgatcct actcccagga gtgtgctcta agttgaaagt agatgcccca ccccctgagt 1980
cagcgccggc aggacttctc accaagccct tctccccctt ttccgctccc tgttcctggt 2040
tcctaggaag cagcccaagg agaagggaaa aggcaggtct gggcaggagg gagcaatgaa 2100
gggcggggca gagggagggc aggagggagg ccggccccct agtaggaaat gagacacagt 2160
agaaataaca ctttataagc ctcttcctcc tcccatctcc tggcctcctt ccatcctcct 2220
ctgcccagac tccgcccctc ccagacggtc ctcacttctc ttttccctag actgcagcca 2280
gcggagcccg cagccggccc gagccaggaa cccaggtccg gagcctcaac ttcaggatgt 2340
tgacaacatt gctgccgata ctgctgctgt ctggctgggc cttttgtagc caagacgcct 2400
cagatggtga gtcgggggca catctcctgc ctcaggatgg ttctggagaa tctcagtcta 2460
tctgggcaca tggcaagacc acaggagagc ttatctcaca gcatctgtgt ctgcagctgg 2520
ctagatctct ctacagggca ggcagagtct tggggactgg ttcgtgtccc aaagccaagg 2580
tgagttagta catttaagcc cctgaaaagg gggagatgaa agaggctagg ggaaacagga 2640
tgactggaaa catgagaaag aaaccagcag agagggtagg agaatcagcc ccagggagag 2700
gggagaaagg ggaactgagg gtgatggtag ataggggtac atctagggga gacgggaaga 2760
ggctcagaag agaagagaaa tggagggaat gggaagaccc tgggaaaact gatggaagaa 2820
gtgggggaag agtggggcag agagaggtta ggggaggcta gggaaaatgg aaggagactg 2880
gtcgcagctg gtggaactgg ggagaaagag atgctgtgcc taatagaact tatgggcgat 2940
caggctactg aagtggccct gtttaagcag aaaagggagt tattaccctc cattataatt 3000
gcacaggggc ctcctttccc ctctctcaca atccccgtaa cttcagtctc cccctcagag 3060
aggcagcaaa taataaccag tattcaatga gtgctcacta tggttaatac atgtattgac 3120
ccatttaact tgcacaaacc cctaaaggtg ggtaatatta ttactatctc cattttatga 3180
ggaggaaact gggtcacaga gtagttaagg accatgtcta gggttatcca taaatatact 3240
tattcacatc tgcagataca aagcacaact tctcaaatgc aaacacagac aggacccact 3300
cacacacaca gatttacaac cccggactca tccaaatgtg ctctgggcat caactctgtg 3360
ccagcctctt ttctgggtgt aggaagcaga gattaccaag catggttcca tagcctagag 3420
gagtccagtg tggcctgtgt gtgtttggag acagccaggt agtatcccgt gagatacaca 3480
ctaatatatg gtggtctggg atcactgaaa cagacacact gtgtctcgtg gggcatcaga 3540
aaaaaatttc caagaagagg gcaactgagc tgggtctttt tttctttgct tttctttctt 3600
ttttcttttc tttttttttt tttttttttg agatggagtc ttgtgctgtc acccaggctg 3660
gaatgcagtg gcacaatttc agctaactgt aacctccaac tcccaggttc aggcgattct 3720
cctgcctcag cctcctgagt agctgggact acaggcatgt accaccacgc ctggctaata 3780
tttgtacttt tagtacagat ggggtttcgc catgttggcc aggctggtct tgaatccctg 3840
acctcaagtg atccgcccgc ctcggcctcc caaagtgctg ggattacagg catgagccac 3900
cgcgcccagt ctctgagctg ggtcttaaat catgaataaa cttcgccagg cagaaaaagg 3960
gaggcagagc aatcctgaca tgctattcat gtgtcagcca aaggcagcat gaggaatccc 4020
aactagtttg atatataagc agcgggaagc ggccagaaaa ggcagcaggg gccaggtctc 4080
tagcagcctt gaatgccagg ctaaagactc tggacttgat cctgtgggga ggcagtgtag 4140
cagaatggct gagtgctgga cttgactgcc tacgtgcaaa ccttggctct gctacactat 4200
ctctgtctca gtttcacatg tagactgggg ttaataatag tagctattgc attaagccac 4260
tggggaaagg cacaaagata ataatgtatg taaagcccat tgcccaggtt ataataagca 4320
ctgaatcgac attggctatg attatttttg attaatgaag gggagggggt tatggcactg 4380
gaagatttta agtaggaaaa ggacatgatc tcatccctgg gtcaggtgga ggtcggaata 4440
gagaacgggg agatgaagta gaaagttact accccagtct agatgagacg gatgaatcct 4500
gaatcagggc agtggaagag gagatggaga acaggcgatg gaattggaat tttattcagg 4560
tcaggatttg ttaaccattt gttccgttgg ttaacaggaa acggggggag ggagagccga 4620

gggtgaaaaa ggaggcagaa aggagtgtct cttccactgc aggcctcagt ttcctcatct 4680
gtaaaacgga gataataatc cctgtcctgt cctcctggca gagttactgt cagcgtcaaa 4740
cgggagaagc ggtgggaggg cacattatag tttatgaagg gtcgagaagg cgggcggcca 4800
gcctcgaggt agggggttat tatcttccgc tgcccgccgc cccctcccac gccggcccag 4860
gctgaagttg actctgcccg caggcctcca aagacttcat atgctccaga tctcctactt 4920
ccgcgacccc tatcacgtgt ggtaccaggg caacgcgtcg ctggggggac acctaacgca 4980
cgtgctggaa ggcccagaca ccaacaccac gatcattcag ctgcagccct tgcaggagcc 5040
cgagagctgg gcgcgcacgc agagtggcct gcagtcctac ctgctccagt tccacggcct 5100
cgtgcgcctg gtgcaccagg agcggacctt ggcctgtgag taggcgcgca gcgggggcgg 5160
ggtctgggcg gggctagtgg gggcggggcc tggcgggtgg gggcggggcc tggcggatgg 5220
aggcgggctg gggcttgcag ggacccggca gccactggag ctcggtggcg cctgggcctt 5280
tgaagattgc tgggtggggg ctggagagag gcagttgtcc ccgctaagaa agccccgact 5340
cgggcggtcg tcctgctggc ataacctctt gggatagacc ctgttggaag gccctgacac 5400
cgtgacgtcg aaggtcccca gaaaactcct cacccctcgc ctcacagtcc tccaactcct 5460
tttcttcata gatctccgtc cttcccttcc cacagccccc agcacttcac cccccaccct 5520
ccagccactt ctcatacaag ctgatgactt cgctcttagc tccactcatg acccgaactc 5580
ttcccccaaa gaccccaagt tcttctctca aagccccact ccttccccgt cacaacccta 5640
actccttctt ctcaaagacc ccaatttctt ttctcaaagc accaagcacc actccgtccc 5700
ccttccccca ccatcatggc ctttaattcc tttctctcct agtcccccac cccaccccct 5760
tttttttttt tttttttttt tttttttgag acggagtctt gctctgtcgt ccaggctgga 5820
gtgcagtggc gcgatctcgg ctcactgcaa cttccgcctc ccgggttcaa gcgattctcc 5880
tgcctcagcc tcccaagcag ctgggactac aggcacccgc caccacgccc ggctaatttt 5940
ttgtattttt agtagagacg gggtttcgcc atgttggcca ggctggtctc gaactcctga 6000
cctcaggcga tccacaagcc tggcctccca aagtgctggg attacaggcg tgagctgccg 6060
cccctgcccc agcctcaccc cctgtttttt ttttctatta cagttgaaca aggcctgaca 6120
attccctttt ttcatcacag tccctggccc cttctttctt agcctctaac aggctaaccc 6180
caaacccctc ctcacagccc caggcccttc tccccatagt tccctgacct agactcccct 6240
ctcctcacag cactgactct tgccttctca tgttcttttc cccttggtgg gcctcgcccc 6300
acacctggca ccctctctgc acagtcccct gatcctgact gtctatccac agttcctctg 6360
accatccgct gcttcctggg ctgtgagctg cctcccgagg gctctagagc ccatgtcttc 6420
ttcgaagtgg ctgtgaatgg gagctccttt gtgagtttcc ggccggagag agccttgtgg 6480
caggcagaca cccaggtcac ctccggagtg gtcaccttca ccctgcagca gctcaatgcc 6540
tacaaccgca ctcggtatga actgcgggaa ttcctggagg acacctgtgt gcagtatgtg 6600
cagaaacata tttccgcgga aaacacgaaa ggtatgatgg gacggggccc aggcctgcaa 6660
gctggggaga gggcgggttc cagacaaatg gatggacctg aaggatggat gcctagagca 6720
acaagaggcc cacagctggg ggtttgggac agaacacacg cagcttcagt cagttggtaa 6780
acgggtccct ttcctctggg gcagaaacgc tttggggttt gactcaaatc atggactcct 6840
tgggggccta ttcttcgggc taactctttg catgttctgc agggagccaa acaagccgct 6900
cctacacttc gctggtcctg ggcgtcctgg tgggcggttt catcattgct ggtgtggctg 6960
taggcatctt cctgtgcaca ggtggacggc gatgttaatt actctccagc cccgtcagaa 7020
ggggctggat tgatggaggc tggcaaggga aagtttcagc tcactgtgaa gccagactcc 7080
ccaactgaaa caccagaagg tttggagtga cagctccttt cttctcccac atctgcccac 7140
tgaagatttg agggagggga gatggagagg agaggtggac aaagtacttg gtttgctaag 7200
aacctaagaa cgtgtatgct ttgctgaatt agtctgataa gtgaatgttt atctatcttt 7260
gtggaaaaca gataatggag ttggggcagg aagcctatgg cccatcctcc aaagacagac 7320
agaatcacct gaggcgttca aaagatataa ccaaataaac aagtcatcca caatcaaaat 7380
acaacattca atacttccag gtgtgtcaga cttgggatgg gacgctgata taatagggta 7440
gaaagaagta acacgaagaa gtggtggaaa tgtaaaatcc aagtcatatg gcagtgatca 7500
attattaatc aattaataat attaataaat ttcttatatt taaggcattg ttatctcctc 7560
cactttgcaa aatttctgga aaagtaacct atacccattt cttctgcttc cttatttctc 7620
actcattctt tttttttttt tttttttttt ttgagacaga gtcttgctct gttgcctagg 7680
ctggagtgca atggtgtgat ctcagctcac tgcaacctct gcctcccggt tcaagcaatt 7740
ctcctgcctc agcctcccaa gcagctggga ttacagatgc atgccaccac acccagctaa 7800
tttttgtatt tttagtagag atggggtttc accacgttgg ccatcctgac ctcgtgatcc 7860
gcctacctcg gcctccccaa gtgctgggat tagacgtgag ccactgcgcc tggtcttctc 7920
actcattctt agacccagtg caatctgact tctctataaa ctactctaag atcaccagta 7980
acctctaatt gtcaaaccgt caccctacat ggtatctgca aatttgcgga ctagaactct 8040
ctttttgcct taacttctga gataccatac ttcaattttt aaaactgttc tgtctacttt 8100
ttttcaatcc ctttgactat gtcatcttac acgattcacc ctggaaatgc tggcttcctt 8160
agaattc 8167

<210> 3
<211> 20
<212> DNA
<213> Artificial Sequence

<220>
<223> Description of Artificial Sequence:PCR primer

<400> 3
gctgaagtgg gcggatcacc                                                    20


<210> 4
<211> 20
<212> DNA
<213> Artificial Sequence

<220>
<223> Description of Artificial Sequence:PCR primer

<400> 4
tctagcctgg gtcatgcggc                                                    20


<210> 5
<211> 20
<212> DNA
<213> Artificial Sequence

<220>
<223> Description of Artificial Sequence:PCR primer

<400> 5
tcttgccgca tgacccaggc                                                    20


<210> 6
<211> 20
<212> DNA
<213> Artificial Sequence

<220>
<223> Description of Artificial Sequence:PCR primer

<400> 6
ggaaggaggc caggagatgg                                                    20


<210> 7
<211> 20
<212> DNA
<213> Artificial Sequence

<220>
<223> Description of Artificial Sequence:PCR primer

<400> 7
ctcttactaa gggtgacgcg                                                    20

<210> 8
<211> 20
<212> DNA
<213> Artificial Sequence

<220>
<223> Description of Artificial Sequence:PCR primer

<400> 8
tctgatgccc cacgagacac                                                    20


<210> 9
<211> 20
<212> DNA
<213> Artificial Sequence

<220>
<223> Description of Artificial Sequence:PCR primer

<400> 9
tctctacagg gcaggcagag                                                    20


<210> 10
<211> 20
<212> DNA
<213> Artificial Sequence

<220>
<223> Description of Artificial Sequence:PCR primer

<400> 10
tcgtggtgtt ggtgtctggg                                                    20


<210> 11
<211> 20
<212> DNA
<213> Artificial Sequence

<220>
<223> Description of Artificial Sequence:PCR primer

<400> 11
aggagtgtct cttccactgc                                                    20


<210> 12
<211> 20
<212> DNA
<213> Artificial Sequence

<220>
<223> Description of Artificial Sequence:PCR primer

<400> 12
cttgtatgag aagtggctgg                                                    20


<210> 13

<211> 21
<212> DNA
<213> Artificial Sequence

<220>
<223> Description of Artificial Sequence:PCR primer

<400> 13
cccagacacc aacaccacga t                                                21


<210> 14
<211> 20
<212> DNA
<213> Artificial Sequence

<220>
<223> Description of Artificial Sequence:PCR primer

<400> 14
gtctgtcttt ggaggatggg                                                  20


<210> 15
<211> 21
<212> DNA
<213> Artificial Sequence

<220>
<223> Description of Artificial Sequence:PCR primer

<400> 15
agaggtggac aaagtacttg g                                                21


<210> 16
<211> 20
<212> DNA
<213> Artificial Sequence

<220>
<223> Description of Artificial Sequence:PCR primer

<400> 16
ggaagccagc atttccaggg                                                  20


<210> 17
<211> 35
<212> DNA
<213> Artificial Sequence

<220>
<223> Description of Artificial Sequence:PCR primer

<400> 17
cctacacttc gctggtcctg ggcgtcctgg tctgc                                 35


<210> 18
<211> 22

<212> DNA
<213> Artificial Sequence

<220>
<223> Description of Artificial Sequence:PCR primer

<400> 18
caagtacttt gtccacctct cc                                                22

## Description

The present invention relates to methods for assessing a genetic risk of thrombosis.

The protein C (PC) system is an important natural anticoagulant mechanism. PC, a vitamin K-dependent zymogen, is activated at the endothelial surface when thrombin binds to thrombomodulin, a protein that transforms the procoagulant enzyme into a potent activator of PC. In the presence of its cofactor, protein S, activated protein C (aPC) inactivates factor Va and Villa, thereby reducing thrombin generation (Dahlback et al., (1995)).

Another factor contributing to protein C activation - endothelial cell activated protein C receptor (EPCR) - was discovered more recently at the surface of endothelial cells (Fukudome et al. (1994)). This receptor, which can bind PC or aPC with the same affinity (Kd = 30 nM), is mainly expressed on endothelial cells of large vessels (Laszik et al. (1997) ; Ye et al. (1999) ; Fukudome et al. (1998)).

Functional studies performed *in vitro* showed a 3- to 5-fold increase in the PC activation rate by the membrane thrombin-thrombomodulin complex when PC is bound to its receptor (Stearns-Kurosawa et al. (1996)). This increase results from a significant effect of EPCR on the Kₘ for PC activation by the thrombin-thrombomodulin complex. Indeed, without EPCR intervention, this Kₘ is significantly higher (1 µM) than the circulating concentration of PC (60-70 nM). By presenting PC to the thrombin-thrombomodulin complex, and owing to its lateral mobility, EPCR reduces the Kₘ and thereby allows the interaction to occur.

In view of these functions, EPCR was expected to intervene in the physiological regulation of coagulation. Evidence for an important role of this type came from baboon studies (Taylor et al. (2000) ; Taylor et al. (2001)), in which an 88% reduction in aPC generation induced by thrombin infusion was observed in animals that had been pretreated with anti-EPCR antibodies blocking the PC/EPCR interaction.

EPCR is a 46-kD type 1 transmembrane glycoprotein homologous to major histocompatibility complex class I/CD1 family proteins (Fukudome et al. (1994) ; Villoutreix et al. (1999) ; Liaw et al. (2001). This 221-amino-acid (aa) protein comprises an extracellular domain, a 25-aa transmembrane domain, and a short (3 aa) intracytoplasmic sequence. The gene is located on chromosome 20 (Hayashi et al. (1999), at position q11.2; it spans 8 kb and comprises 4 exons (Hayashi et al. (1999) ; Simmonds et al. (1999). The first exon encodes the 5' untranslated region and the signal peptide, exons 2 and 3 most of the extracellular domain, and exon 4 the remaining parts of the protein and the 3' untranslated region. The proximal part of the promoter was recently functionally characterized (Rance et al. (2003).

Several authors have reported the presence in plasma of a soluble form of EPCR (sEPCR) (Kurosawa et al. (1997) ; Kurosawa et al. (1998)) that probably lacks the transmembrane domain and cytoplasmic tail. This sEPCR is detected as a single species of 43 kD, resulting from shedding of membrane EPCR by the action of a metalloprotease (Xu et al. (2000)) which is stimulated by thrombin and by some inflammatory mediators (Gu et al. (2000)). Soluble EPCR binds PC and aPC with similar affinity (Fukudome et al. (1996) ; Regan et al. (1996)), but its binding to aPC inhibits the anticoagulant activity of aPC by blocking its binding to phospholipids and by abrogating its ability to inactivate factor Va (Liaw et al. (2000)). By contrast with the membrane-associated form of EPCR, PC binding to sEPCR does not result in enhanced aPC generation by the thrombin-thrombomodulin complex (Regan et al. (1996)). A recombinant sEPCR has recently been crystallized (Oganesyan et al., 2002).

Dysfunctional EPCR-dependent activation of PC would potentially be thrombogenic. A loss of function could result from mutations leading to decreased expression of membrane EPCR. A 23-bp insertion has been reported to impair EPCR functions by leading to the synthesis of a truncated protein that is not expressed on endothelial surfaces (Biguzzi et al. (2001)). Although initially identified in thrombophilic subjects (von Depka et al. (2001 )), the role of this mutation in thrombosis is difficult to assess because its allelic frequency is low (von Depka et al. (2001) ; Akar (2002) ; Poort et al. (2002) ; Galligan et al. (2002)). Point mutations were recently described within the promoter region (Biguzzi et al. (2002)) of the gene in four thrombophilic subjects, but the involvement of these mutations in gene regulation could not be clearly demonstrated.

Another possible mechanism leading to dysfunction of the EPCR-mediated coagulation-regulating mechanism consists of mutations (or polymorphisms) leading to increased levels of sEPCR. Indeed, increased sEPCR levels may be prothrombotic, as sEPCR can inhibit aPC activity, as well as PC activation, by competing for PC with membrane-associated EPCR.

Two recent studies show that sEPCR levels vary widely among healthy subjects (Stearns-Kurosawa et al. (2002) ; Stearns-Kurosawa et al. (2003). While sEPCR levels are between 75 and 178 ng/mL in 80% of subjects, the remaining 20% of subjects have values between 200 and 700 ng/mL. This bimodal distribution has repeatedly been reported in both French and Italian populations (Stearns-Kurosawa et al. (2003)).

Several polymorphisms in the EPCR have been studied for possible association with the risk of thrombosis.

G/A polymorphism affecting nt 6936 has been described in two different studies, both of which showed a very similar frequency of the G allele in thrombophilic and control Caucasian subjects (Espana et al 2001, wherein the polymorphism was designated A7685 ; Poort et al 2002, wherein the polymorphism was designated A4300G).

The 7014 G/C polymorphism has also been reported in three studies (Espana et al 2001, Galligan et al 2002 and Medina et al 2003) wherein the polymorphism was designated G7763C, G5252C and G4678C, respectively.

Finally, a third polymorphism corresponding to nt 4868 has been described by Poort et al (2002) with T3997C numbering.

In the XVIIIth Congress of the International Society on Thrombosis and Haemostasis (July 2001), Espana et al reported that an EPCR allele bearing the 4678 C polymorphism (that corresponds to nt 7014 polymorphism described here) has a protective effect against thrombosis, and at the XIX Congress of the International Society on Thrombosis and Haemostasis (July 2003), Navarro et al described a similar effect of this allele in thrombophilic carriers of the factor V Leiden mutation.

The inventors now extensively analysed the EPCR gene and identified several polymorphisms that were in complete linkage disequilibrium, defining three haplotypes (designated A1, A2 and A3). One of three haplotypes was associated with increased sEPCR levels, offering the first evidence that inter-individual variations in sEPCR levels are genetically regulated.

As sEPCR can inhibit both aPC generation and aPC activity, the inventors examined whether the haplotype associated with high sEPCR levels carried an increased risk of venous thrombosis. On comparing 338 subjects with thrombosis and 338 age- and sex-matched healthy controls, the inventors observed a significantly higher allelic frequency of this haplotype in the cases.

Based on these results, the present invention provides a method for distinguishing A1, A2 and A3 haplotypes in the EPCR receptor, the latter being associated with a higher risk to develop thrombosis.

This method is thus useful to determine the risk of developing thrombosis, especially venous thrombosis, in a subject.

### EPCR receptor

As above mentioned, the human EPCR receptor has been cloned. EPCR has also been characterized in other species, such as mouse and bovine :

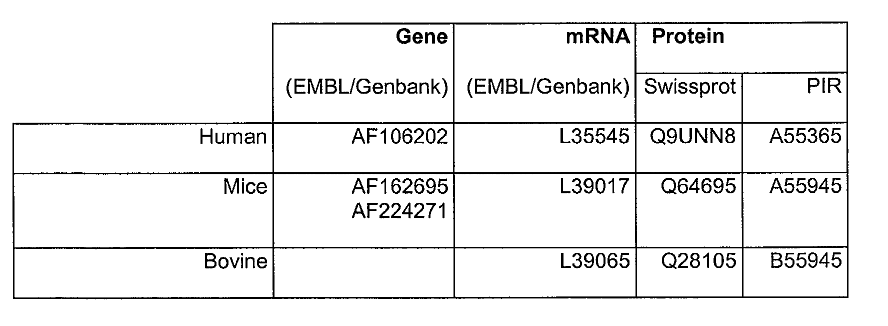

In the context of the present invention, the term *"EPCR"* or *"EPCR* receptor" refers to the EPCR receptor of any species, especially in human, but also in other mammals or vertebrates to which the methods of the invention can apply, if desired. The term *"subjecf"* thus refers to any human patient or any mammal or vertebrate.

The human EPCR gene sequence is available on Genbank (access number: AF106202), and is also shown in SEQ ID NO:1 (A2 haplotype). In this sequence, the starting codon for the open reading frame is located at nucleotide 2337.

A chromosomal sequence is also available on Genbank (AL 356652), and corresponds to the A1 haplotype. In that sequence, the EPCR gene is located from nucleotide 29655 to nucleotide 37818.

A sequence of the A3 haplotype is represented as SEQ ID No: 2.

### Haplotypes

The inventors found various nucleotide polymorphisms (SNPs) in the EPCR gene. In the present invention, these polymorphisms are sometimes referred to as "the polymorphic positions of interest".

To make it simple, these polymorphisms are herein numbered according to SEQ ID No:1, starting from nucleotide 1.

However it should be noted that this numbering is arbitrary.

The identified SNPs defined three haplotypes, that were designated A1, A2 and A3.

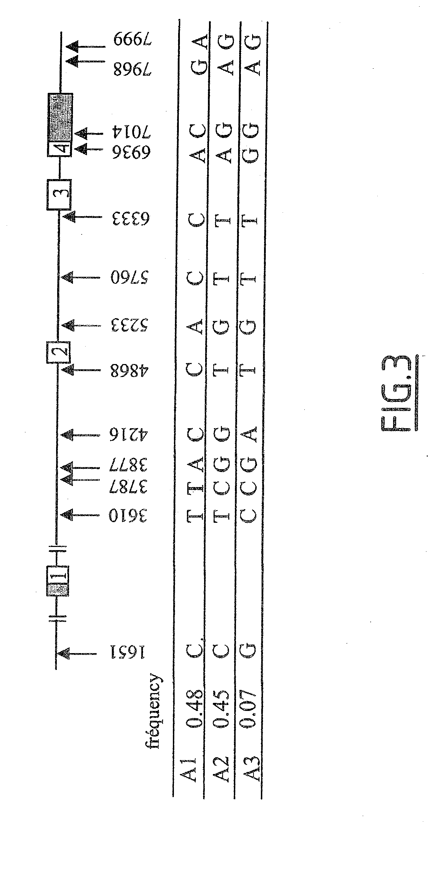

As shown in figure 3, A1 and A2 were major haplotypes, with allelic frequencies of 0.48 and 0.45, respectively. The A1 haplotype consisted of a combination of T at nt 3787, A at nt 3877, C at nt 4216, C at nt 4868, A at nt 5233, C at nt 5760, C at nt 6333, C at nt 7014, G at nt 7968 and A at nt 7999. The A2 haplotype was a combination of C at nt 3787, G at nt 3877, G at nt 4216, T at nt 4868, G at nt 5233, T at nt 5760, T at nt 6333, G at nt 7014, A at nt 7968 and G at nt 7999. A3, the least common haplotype (allelic frequency 0.07), differs from A2 haplotype at four nucleotide positions (G at nt 1651, C at nt 3610, A at nt 4216 and G at nt 6936).

The A3 haplotype is thus defined as the simultaneous presence of the following polymorphisms :
- G at position 1651
- C at position 3610
- A at position 4216
- G at position 6936
- C at position 3787
- G at position 3877
- T at position 4868
- G at position 5233
- T at position 5760
- T at position 6333
- G at position 7014
- A at position 7968
- G at position 7999

The isolated nucleic acid of the EPCR receptor gene with the A3 haplotype is also part of the present invention.

A subject of the invention is thus an isolated nucleic acid encoding the EPCR receptor, which nucleic acid comprises the EPCR gene sequence with the simultaneous presence of :
- G at position 1651
- C at position 3610
- A at position 4216
- G at position 6936
- C at position 3787
- G at position 3877
- T at position 4868
- G at position 5233
- T at position 5760
- T at position 6333
- G at position 7014
- A at position 7968
- G at position 7999

Such nucleic acid preferably comprises SEQ ID No: 2.

The identification of any of :
- G at position 1651
- C at position 3610
- A at position 4216
- G at position 6936

is sufficient to identify the A3 haplotype.

### Thrombosis risk

The present invention provides an *in vitro* method for determining the risk of developing thrombosis in a subject, which method comprises identifying polymorphisms of EPCR gene on at least one of positions 1651, 3610, 4216, and 6936, wherein the presence of G at position 1651, C at position 3610, A at position 4216, or G at position 6936 is indicative of a higher risk to develop thrombosis in comparison with a control subject that does not show the same polymorphisms.

In a preferred embodiment, the method comprises identifying polymorphisms of EPCR gene at positions 1651, 3610, 4216, 6936, 3787, 3877, 4868, 5233, 5760, 6333, 7014, 7968, 7999 of SEQ ID No: 1, wherein the simultaneous presence of :
- G at position 1651
- C at position 3610
- A at position 4216
- G at position 6936
- C at position 3787
- G at position 3877
- T at position 4868
- G at position 5233
- T at position 5760
- T at position 6333
- G at position 7014
- A at position 7968
- G at position 7999
are designated A3 haplotype and, when present on at least one allele, are indicative of a higher risk to develop thrombosis in comparison with a control subject without any A3 allele.

A higher risk to develop thrombosis means a significantly greater risk in carriers of the A3 allele than in non carriers.

Such a relative risk is frequently estimated through case-control studies and the use of the odds ratio (OR). An OR of 1.1 corresponds to an increase of 10% of the risk of developing disease and an OR of 2 corresponds to a 100 % increased risk.

The risk of developing thrombosis is connected with increased plasma levels of soluble EPCR.

In the context of the present invention, the term "thrombosis" means formation of a clot in a blood vessel or in a heart cavity.

In a preferred embodiment, venous thrombosis is encompassed.

Consequently, the identification of the A3 haplotype of the EPCR receptor in a subject is further indicative of a higher risk to develop thromboembolic diseases, e.g. pulmonary embolism or deep venous thrombosis (DVT).

For instance, knowing the genetic status of an individual with respect to the EPCR haplotype is particularly useful to prevent recurrence of lung embolisms, that are fatal in 20 % of cases.

Within the meaning of the invention, "thrombosis" as above defined encompasses other thrombotic states such as arterial thrombosis as well as thrombotic microangiopathy or intravascular disseminated coagulation.

### Identification of the presence of the A3 allele

The methods described above for determining the thrombosis risk involve the identification of the A1, A2 or A3 haplotype of the EPCR gene.

Such identification may be performed by any technique well known from the person skilled in the art.

In practicing the methods of the invention, an individual's polymorphic pattern with regard to the haplotype of interest can be established by obtaining DNA from the individual and determining the sequence at the polymorphic positions of the EPCR receptor gene interest.

The DNA may be obtained from any cell source. Non-limiting examples of cell sources available in clinical practice include blood cells, buccal cells, cervicovaginal cells, epithelial cells from urine, fetal cells, hair, or any cells present in tissue obtained by biopsy, e.g. tumor biopsy. Cells may also be obtained from body fluids, including without limitation blood, saliva, sweat, urine, cerebrospinal fluid, feces, and tissue exudates at the site of infection or inflammation. DNA may be extracted from the cell source or body fluid using any of the numerous methods that are standard in the art. It will be understood that the particular method used to extract DNA will depend on the nature of the source. In another embodiment, the analysis is achieved without extraction of the DNA, e.g. on whole blood (Mercier et al, 1990).

Determination of the sequence of the DNA at the polymorphic positions of the A1, A2 and A3 haplotype is achieved by any means known in the art. Numerous strategies for genotype analysis are available (Antonarakis et al., 1989 ; Cooper et al., 1991 ; Grompe, 1993). The strategies include, but are not limited to, direct sequencing, restriction fragment length polymorphism (RFLP) analysis, hybridization with allele-specific oligonucleotides, allele-specific PCR, PCR using mutagenic primers, ligase-PCR, HOT cleavage, denaturing gradient gel electrophoresis (DGGE), temperature denaturing gradient gel electrophoresis (TGGE), single-stranded conformational polymorphism (SSCP) and denaturing high performance liquid chromatography (Kuklin et al., 1997). Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology (see e.g. O-Donnell-Maloney et al., 1996); and real-time quantitative PCR. Preferably, DNA from a subject is first subjected to amplification by polymerase chain reaction (PCR) using specific amplification primers. However several other methods are available, allowing DNA to be studied independently of PCR, such as the oligonucleotide ligation assay (OLI), rolling circle amplification (RCA) or the Invader™assay.

In a particular embodiment of the invention, it is provided an in vitro method for identifying at least one polymorphism of an haplotype of the EPCR receptor associated with thrombosis in a subject, which method comprises analyzing genomic DNA of a biological sample, in at least one of the regions of the EPCR gene, located around positions 1651, 3610, 4216 and 6936, preferably as well as in the regions of the EPCR gene located around positions 3787, 3877, 4868, 5233, 5760, 6333, 7014, 7968 and 7999 of SEQ ID No: 1;
wherein the presence of :
- G at position 1651
- C at position 3610
- A at position 4216, or
- G at position 6936, more particularly the simultaneous presence of these SNPs in combination with the following :

- C at position 3787
- G at position 3877
- T at position 4868
- G at position 5233
- T at position 5760
- T at position 6333
- G at position 7014
- A at position 7968, and
- G at position 7999
when present on at least one allele, are indicative of a higher risk to develop thrombosis, in comparison with a control subject.

The analysis may preferably comprise a step of amplification (e.g. by PCR) of said regions of the genomic DNA.

In a particular embodiment the analysis is undertaken on isolated (*i*.*e*. extracted) genomic DNA.

A preferred technique for identifying the polymorphisms of the EPCR receptor includes sequencing or RFLP analysis.

To identify the G6936 polymorphism, one can for instance create a restriction site for the endonuclease *Pstl* by amplification with mutagen primers, when the amplified fragment contains an A at position 6936, which corresponds to haplotype A1 or A2, so that when the amplified fragment contains a G, which corresponds to haplotype A3, it remains undigested.

### Kits

According to an aspect of the invention, the A3 haplotype is detected by contacting the DNA of the subject with a nucleic acid probe, that is optionally labeled.

Primers may also be useful to amplify or sequence the portion of the EPCR gene containing the polymorphic positions of interest.

Such probes or primers are nucleic acids that are capable of specifically hybridizing with a portion of the EPCR gene sequence containing the polymorphic positions of interest. That means that they are sequences that hybridize with the nucleic acid sequence to which it refers under conditions of high stringency (Sambrook et al, 1989). These conditions are determined from the melting temperature Tm and the high ionic strength. Preferably, the most advantageous sequences are those which hybridize in the temperature range (Tm - 5°C) to (Tm - 30°C), and more preferably (Tm - 5°C) to (Tm - 10°C). A ionic strength of 6xSSC is more preferred. For instance, high stringency hybridization conditions correspond to the highest Tm, e.g., 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridization with shorter nucleic acids, i.e., oligonucleotides, the position of mismatches becomes more important, and the length of the oligonucleotide determines its specificity (see Sambrook et al., supra, 11.7-11.8). A minimum length for a hybridizable nucleic acid is at least about 10 nucleotides ; preferably at least about 15 nucleotides ; and more preferably the length is at least about 20 nucleotides.

Preferred probes or primers are described in the legend to the figures or in the examples below.

When the primers are mutagenic primers, the above rules for determining the hybridisation conditions are to be adapted. The sequences of mutagenic primers may indeed partially vary from the wild-type sequence, so as to introduce restriction site when a given allele is amplified. Furthermore such mutagenic primers are frequently 30 to 40 nucleotide-long.

The present invention further provides kits suitable for determining at least one of the polymorphism of the A3 haplotype of the EPCR gene.

The kits may include the following components :
(i) a probe as above defined, usually made of DNA, and that may be pre-labelled. Alternatively, the probe may be unlabelled and the ingredients for labelling may be included in the kit in separate containers ; and
(ii) hybridization reagents : the kit may also contain other suitably packaged reagents and materials needed for the particular hybridization protocol, including solid-phase matrices, if applicable, and standards.

In another embodiment, the kits may include :
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

In a particular embodiment, it is provided a kit which comprises a pair of nucleotide primers specific for amplifying all or part of the EPCR gene comprising at least one of the positions of the SNPs that are identified herein, especially positions 1651, 3610 and 4216 of SEQ ID No:1 (or SEQ ID No: 2).

The below figures and examples illustrate the invention without limiting its scope.

### LEGENDS TO THE FIGURES:

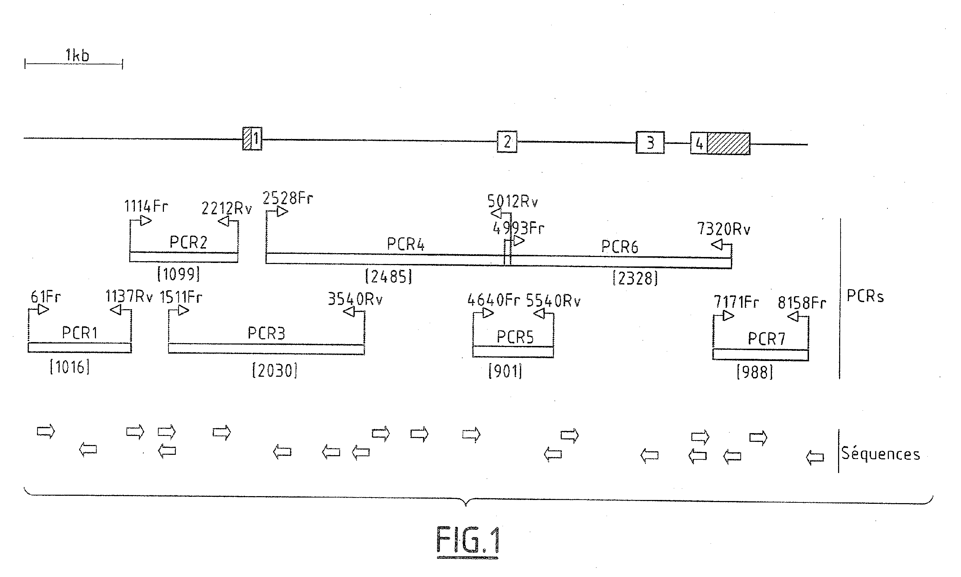

Figure 1 shows the location of the amplification and sequencing primers on the EPCR gene.

Exons are symbolized by boxes. The 5' part of exon 1 and the 3' part of exon 4, which are noncoding, are striped.

The primer pairs used to amplify nearly the entire gene in seven PCR runs are indicated, and the size of the amplification products (in base pairs) is indicated between brackets. The oligonucleotides are numbered according to the position of their 5' nucleotide on sequence AF 106202 (Genbank accession number, (SEQ ID No:1)), followed by Fr for sense primers or Rv for antisense primers. The sequences of the amplification primers are indicated below, from 5' to 3':

PCR1: 61Fr GCTGAAGTGGGCGGATCACC (SEQ ID No:3) and 1137Rv
TCTAGCCTGGGTCATGCGGC(SEQ ID No:4)

PCR2:1114Fr TCTTGCCGCATGACCCAGGC (SEQ ID No:5) and 2212Rv
GGAAGGAGGCCAGGAGATGG (SEQ ID No:6)

PCR3: 1511Fr CTCTTACTAAGGGTGACGCG (SEQ ID No:7) and 3540Rv
TCTGATGCCCCACGAGACAC (SEQ ID No:8)

PCR4: 2528Fr TCTCTACAGGGCAGGCAGAG (SEQ ID No:9) and 5012Rv
TCGTGGTGTTGGTGTCTGGG (SEQ ID No:10)

PCR5: 4640Fr AGGAGTGTCTCTTCCACTGC (SEQ ID No:11) and 5540Rv
CTTGTATGAGAAGTGGCTGG (SEQ ID No:12)

PCR6: 4993Fr CCCAGACACCAACACCACGAT (SEQ ID No:13) and 7320Rv
GTCTGTCTTTGGAGGATGGG (SEQ ID No:14)

PCR7:7171Fr AGAGGTGGACAAAGTACTTGG (SEQ ID No:15) and 8158Rv
GGAAGCCAGCA TTTCCAGGG (SEQ ID No:16)

Figure 2A shows the distribution of sEPCR levels in 100 healthy male volunteers.

Figure 2B shows the concordance of sEPCR levels at two visits.

Figure 3 represents the three EPCR gene haplotypes :

The 13 polymorphisms found to be in complete linkage disequilibrium defined three haplotypes designated A1, A2 and A3. The nucleotides are numbered according to the Genbank sequence (accession number AF1 06202, SEQ ID No:1).

Figure 4 represents the plasma sEPCR levels according to the genotype in 100 healthy male volunteers.

The mean±S.D. (n, range ; median) were 79.4±16.6 ng/mL (20, 56.4-108.2; 79.5), 84.6±16.3 (48, 50.5-126; 82.9), 314±218.7 (8, 211.3-854.4;235.1), 85.5±20.3 (18, 63.3-137.5; 77.8) and 196.7±46.2 ng/mL (6, 138.4-266.55; 190.5) for A1 A1, A1 A2, A1 A3, A2 A2 and A2 A3 subjects, respectively. When excluding the subject with atypical high sEPCR levels (854 ng/mL), mean ± S.D. (n, range ; median) of A1A3 subjects were 237.9±20.2 ng/mL (7, 211.3-274.9; 233.05).

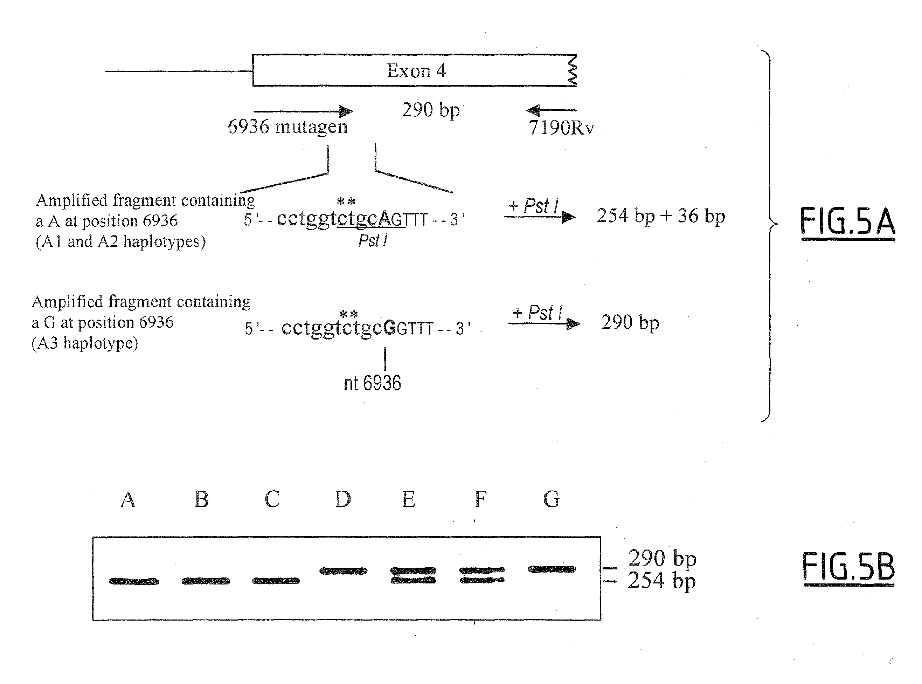

Figures 5A and 5B represent the rapid A3 haplotype identification method.

Figure 5A : Schematic representation of the part of the human EPCR gene exon 4 containing G 6936, which identifies the A3 haplotype. The 6936 mutagen primer contains two foreign nucleotides at positions n-4 and n-3 from the 3' end (indicated by asterisks) in order to create a restriction site for the endonuclease *Pst I* when the amplified fragment contains an A at position 6936, which corresponds to haplotype A1 or A2; the amplified fragment containing a G, which corresponds to haplotype A3, remains undigested. After genomic amplification using this primer and the 7190Rv primer, the PCR-amplified fragment contains a Pst I site (CTGCA/G; underlined) when nucleotide 6936 is an A. In the amplified fragment, the part corresponding to the primer is shown in lower letters.

Fiaure 5B : 2% agarose gel electrophoresis of digested PCR products obtained using 6936 mutagen and 7190Rv primers. Lane A to C: subjects homozygous for an A at position 6936 (A1/A1, A2/A2 and A1/A2, respectively); a restriction site for *Pst I* was created, allowing the amplified fragment to be completely digested into two fragments of 254 and 36 bp (the latter is not visible on the gel). Lane D: subject homozygous for a G at position 6936 (A3/A3): no *Pst I* restriction site is available, and the fragment remains undigested at 290 bp. Lanes E and F: subjects heterozygous A/G at position 6936 (A1/A3 or A2/A3, i.e. A3 "heterozygotes"): both patterns are visible, corresponding to the undigested (290 bp) and digested (254 bp) amplified fragments. Lane G: Undigested PCR-amplified fragment.

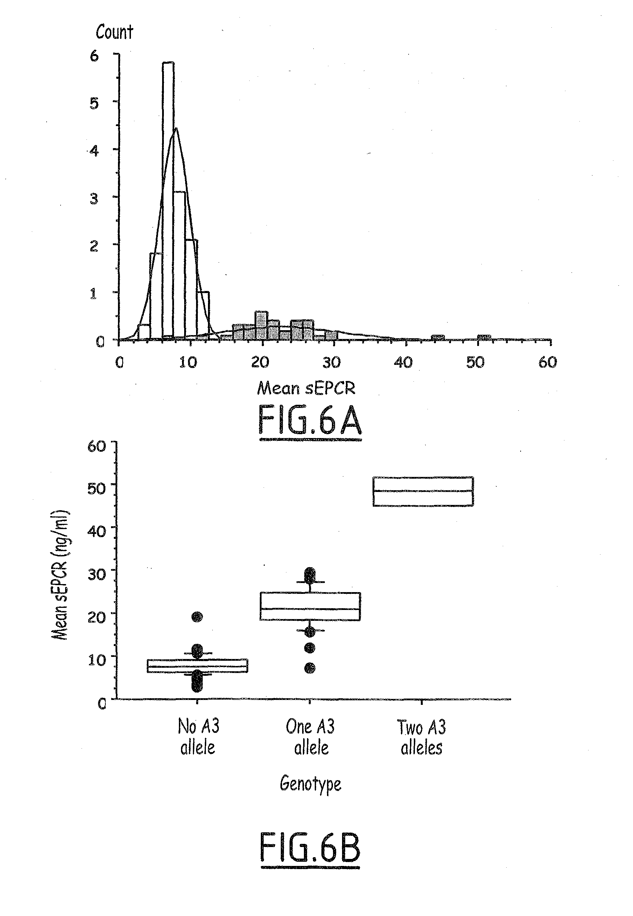

Figure 6A and 6B represent the plasma sEPCR levels according to the presence or absence of A3 alleles, in a series of 176 healthy female controls from the PATHROS case-control study.

Figure 6A : Distribution of sEPCR levels

Figure 6B : Correlation between the genotype and the sEPCR level.

### EXAMPLE : Identification of a haplotype of the EPCR gene that is associated with increased plasma levels of sEPCR and is a candidate risk factor for thrombosis

### Subjects, Materials and Methods

### - Materials

Evacuated tubes were from Beckton Dickinson (Le Pont de Claix, France). The Qiamp Maxi kit was from Qiagen (Courtaboeuf, France). The sEPCR Asserachrom kit was kindly supplied by Stago Laboratories (Asnières, France). The DNA sequencing kit (Big Dye Terminator V3.0 Cycle Sequencing Ready Reaction with AmpliTaq DNA Polymerase FS) and the ABI Prism 3700 sequencer were from Applied Biosystems (Applera, Courtaboeuf, France). The dNTP mix was from Amersham Biosciences Europe (Orsay, France). Oligonucleotides were from Proligo (Paris, France). Plates for amplified-product purification were from Millipore (Saint-Quentin en Yvelines, France). *Pst I* restriction endonuclease was from New England Biolabs (Ozyme, Saint Quentin en Yvelines, France). Agarose was from Life Technologies (Invitrogen, Cergy-Pontoise, France).

### - Healthy subjects and patients

One hundred unrelated healthy Caucasian male volunteers aged from 18 to 35 years were recruited and studied at the Clinical Investigations Center of Hôpital Européen Georges Pompidou. This population has been described in detail elsewhere (Fontana et al. (2003) ; Dupont et al. (2003)). Briefly, the volunteers were non smokers and had not taken any medication for at least 10 days before blood sampling. Volunteers with a personal or family history of excessive bleeding or thrombosis were excluded. The subjects underwent a physical examination and routine laboratory tests ((Fontana et al. (2003) ; Dupont et al. (2003)), including C-reactive protein and F1+2 assay.

Blood was collected from all volunteers by venipuncture in tubes containing 0.11 M sodium citrate (1 vol/9 vol) on day 1 (visit 1) and day 7 (visit 2). Plasma was obtained by centrifugation at 2300 g for 20 min, and was immediately subjected to routine laboratory tests or stored at -80°C until use. Genomic DNA was isolated from peripheral blood mononuclear cells by using the Qiamp Maxi kit according to the manufacturer's instructions.

A group of 338 patients, matched for age and sex with 338 controls, were studied in a second phase. These subjects had participated in a case-control study, the PAris THRombosis Study (PATHROS), designed to seek genetic risk factors for venous thromboembolism (VTE). The inclusion and exclusion criteria applied to cases, and their clinical and biological characteristics, have been extensively described elsewhere (Arnaud et al. (2000)). Briefly, the patients had had at least one episode of objectively diagnosed deep venous thrombosis (documented by compression and ventilation lung ultrasonography or venography) and/or pulmonary embolism (documented by perfusion and ventilation lung scanning, convention pulmonary angiography, or computed tomographic angiography). The controls were healthy European subjects recruited from a healthcare center to which they had been referred for a routine checkup. Subjects with a history of VTE, arterial disease or known malignancy were excluded on the basis of a medical questionnaire. To avoid a possible bias due to the fact that controls came from a health care center, the inventors checked that the frequencies of factor V and Prothrombin G20210A mutations were similar to that observed in other control populations (Arnaud et al., 2000; Emmerich et al., 2001).

The study protocols were approved by local ethics committee.

Blood was collected and plasma prepared as previously described (Arnaud et al. (2000). DNA was extracted from white blood cells by using a standard method (Miller et al. (1998). Factor V Arg506Gln and prothrombin gene G20210A mutations were identified as previously described (Alhenc-Gelas et al. (1999). All samples were obtained with the participants' informed consent.

### - Soluble EPCR assay

Soluble EPCR (sEPCR) levels were determined in plasma by using sEPCR Asserachrom ELISA kits from the same batch, according to the manufacturer's instructions.

### - EPCR gene screening for polymorphisms

The nucleotides of the EPCR gene were numbered according to the sequence available under GenBank accession number AF106202 (SEQ ID No:1). As shown in figure 1, seven amplification fragments spanned more than 99% of the EPCR gene. The location of the amplification primers relative to the EPCR gene sequence, as well as their nucleotide sequences, are indicated in figure 1 and its legend. Each amplification product was purified by filtration on Millipore plates and sequenced with a DNA sequencing kit according to the manufacturer's instructions; the sequencing products were analyzed on ABI Prism 3700 sequencer.

Screening of 40 subjects from a given population is sufficient to identify polymorphisms having a frequency of 5% or more, with a confidence interval (Cl) of 95%. Thus, the entire EPCR gene of the first 48 consecutive healthy volunteers was screened for polymorphisms as described above. Then, the polymorphic sites identified in these 48 subjects were screened for in 52 additional healthy subjects, with primers targeting these sites.

### - Haplotype A3 identification in the PATHROS population.

A rapid method of A3 haplotype identification was developed by using the G at nt 6936 of the EPCR gene as marker. The region surrounding nucleotide 6936 was amplified by using a mutagenic 35-mer 6936 mutagen (5'-CCTACACTTCGCTGGTCCTGGGCGTCCTGGTctGC-3' (SEQ ID No:.17) as upstream primer, and a 22-mer 7190Rv (5'-CAAGTACTTTGTCCACCTCTCC-3' (SEQ ID No: 18) as downstream primer. The upstream primer bore two foreign nucleotides (underlined lowercase characters in the preceding sequence), thereby allowing amplified fragments bearing an A at position 6936 of the EPCR gene (thus corresponding to haplotype A1 or A2) to be cleaved by the restriction endonuclease Pst I, whereas amplified fragments bearing a G (and corresponding to haplotype A3) remained undigested. Twenty microliters of the 290-bp amplification product was incubated overnight at 37°C with 20 units of *Pst I*, and digestion was checked by electrophoresis on 2% agarose gel.

### - Statistical analysis

Continuous variables are reported as means and standard deviation or as medians and range (according to their distribution), and categorical variables are reported as counts and percentages. Skewed variables were log-transformed before analysis. Individual subjects' s EPCR plasma concentrations at visits 1 and 2 were compared using a concordance test (Lin (1989)). The chi-square test was used to compare the observed genotype frequencies with the Hardy-Weinberg equilibrium prediction. The association between the genotype and the biological phenotype (sEPCR level) was tested using ANOVA. Comparisons between case and control subjects were based on Student's unpaired t-test for continuous variables, and the chi-square test or Fisher's exact test for categorical variables. Multivariate analysis was used to determine the odds ratio, based on multiple logistic regression. Statistical tests were run on Statview5® statistical software (SAS), and differences with P values <0.05 were considered statistically significant.

### Results

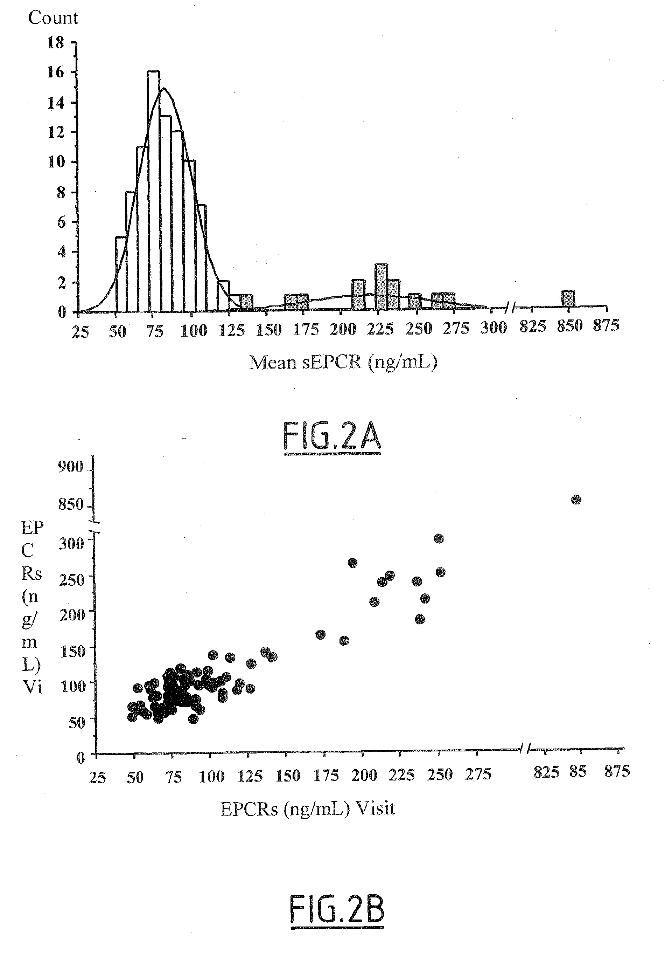

To assess the intra-individual variability of plasma sEPCR levels, the inventors tested two blood samples, obtained one week apart, from each of 100 healthy male volunteers, except for two subjects who did not attend visit 2. At least two phenotypic groups were identified. Values in both groups had a gaussian distribution (Figure 2A); sEPCR levels were below 137.5 ng/ml (3 nM) at both visits in 84 subjects, and above 138.5 ng/ml at both visits in 14 subjects. Plasma sEPCR levels concorded between the two visits (R² = 0.95, P< 0.0001 )(Figure 2B). One subject had a very high sEPCR level (854 ng/mL) at both visits. As plasma sEPCR levels may be influenced by inflammation, CRP levels were also determined in all the subjects. The results (CRP values always below 5 mg/mL) ruled out a role of inflammation in the bimodal distribution of sEPCR levels. F1+2 levels in the 84 subjects with lower sEPCR levels (median 1.03 nM; range 0.56-3.45) were similar to those in the 14 subjects with higher sEPCR levels (median 1.28; range 0.68-3.12) (P=0.57), ruling out elevated thrombin generation in the latter group.

The existence of different phenotypic groups of sEPCR expression, together with the stability of individual levels over time, pointed to genetic control of the sEPCR level. The inventors therefore analyzed the EPCR gene in 48 consecutive healthy volunteers, from nucleotide 80 to 8100 (*i.e.* 99% of the available AF106202 sequence (SEQ ID No:1), corresponding to ~2300 nucleotides upstream of the ATG codon, the exons, the introns and ~1500 nt downstream of the stop codon). The inventors found 16 single nucleotide polymorphisms (SNP) located throughout the gene. The first was a C to G transversion of nucleotide (nt) 1651, located within the promoter region. Six other SNPs were located in intron 1 and affected nt 3610 (T to C transition), nt 3787 (T to C transition), nt 3877 (A to G transition), nt 4216 (C, G or A), nt 4414 (T to C transition) and nt 4868 (C to T transition). Four SNPs were located in intron 2, and affected nt 5233 (A to G transition), nt 5760 (C to T transition), nt 6146 (G to A transition) and nt 6333 (C to T transition). Exon 4 contained two SNPs, with an A to G transition at nt 6936, changing Ser 219 to Gly (Simmonds et al. (1999)), and a C to G transversion at nt 7014, in the non coding part of exon 4. Finally, three SNPs were located in the 3' untranslated (3'-UTR) part of the gene; they consisted of a C to G transversion at nt 7966, a G to A transition at nt 7968 and an A to G transition at nt 7999. These polymorphisms had allelic frequencies above 0.05, except for 4414C and 6146A (0.041 and 0.036, respectively).

Using primers targeting the 14 frequent polymorphic positions, the inventors amplified and sequenced the corresponding regions of the EPCR gene in the other 52 healthy volunteers in order to determine the allelic frequencies of the polymorphisms. All but one (nt 7966) of the 14 frequent SNPs were in complete linkage disequilibrium. These 13 SNPs defined three haplotypes, that were designated A1, A2 and A3. As shown in figure 3, A1 and A2 were major haplotypes, with allelic frequencies of 0.48 and 0.45, respectively. The A1 haplotype consisted of a combination of T at nt 3787, A at nt 3877, C at nt 4216, C at nt 4868, A at nt 5233, C at nt 5760, C at nt 6333, C at nt 7014, G at nt 7968 and A at nt 7999. The A2 haplotype was a combination of C at nt 3787, G at nt 3877, G at nt 4216, T at nt 4868, G at nt 5233, T at nt 5760, T at nt 6333, G at nt 7014, A at nt 7968 and G at nt 7999. A3, the least common haplotype (allelic frequency 0.07), differs from A2 haplotype at four nucleotide positions (G at nt 1651, C at nt 3610, A at nt 4216 and G at nt 6936). The allelic frequencies of the three haplotypes were in Hardy-Weinberg equilibrium (χ2 test, p>0.05).

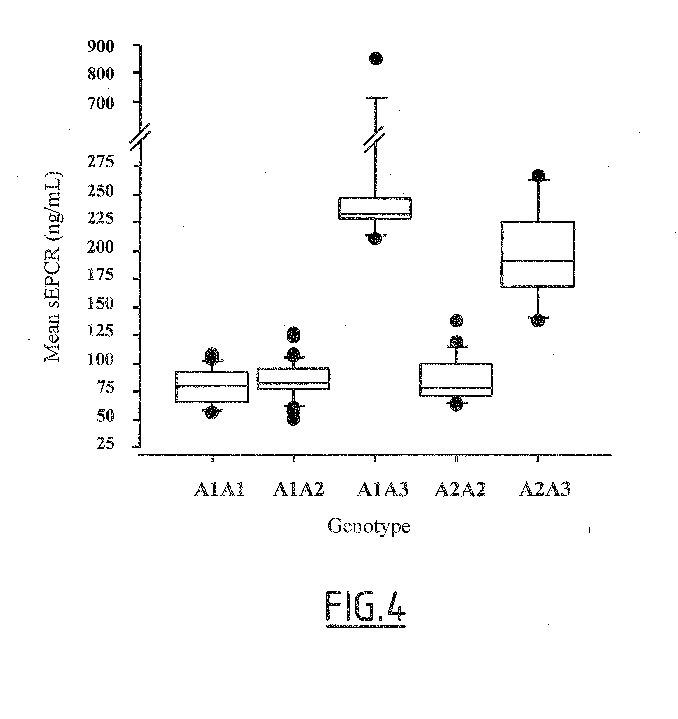

To establish whether or not the plasma sEPCR level is genetically regulated, the inventors compared the plasma sEPCR level with the sEPCR genotype (Figure 4). As plasma sEPCR levels were stable between the two visits, we used the mean value for each subject. As shown in figure 4, sEPCR levels were significantly higher in subjects carrying one A3 allele (A1 A3 or A2 A3) than in subjects carrying no A3 allele. No significant difference in sEPCR levels was observed between A1 A1 or A2 A2 homozygotes and A1 A2 heterozygotes. The mean sEPCR level in subjects having at least one A3 allele was 264±174ng/ml (range 138.5 to 854) [218.9 ± 39.36 ng/ml (range: 138.5 to 274.9) after excluding the subject with a value of 854 ng/mL], compared to 83.6 ± 17.2 ng/ml (range: 50.5 to 137.5) in the other subjects (p<0.0001, 95% CI). It is important to underline that all the subjects carrying the A3 haplotype had elevated sEPCR levels at both visits, one week apart. Interestingly, none of the 100 volunteers was an A3 A3 homozygote.

To evaluate the possible influence of sEPCR levels on the risk of venous thromboembolism, the inventors investigated a cohort of 338 patients matched for age and sex with 338 healthy controls from the PATHROS study. The patients were 162 men (47.9%) and 176 women (52.1 %). Mean age was 46 ± 13 years in the control group and 48 ± 15 years in the patient group (P=0.06). The main characteristics and clinical events of the patients and controls are shown in Table 1 :

**Table 1.**

| Characteristics of the PATHROS case-control study population | | | |
|---|---|---|---|
| Sex ratio (men/women) | Cases (n=338) 162/178 | Controls (n=338) 162/178 | *P* |
| Age (years) | 48 ± 15 | 46 ± 13 | 0.06 |
| OC or HRT in females, % | 28.9 | 40.2 | |
| Pulmonary embolism, % | 43.5 | .... | |
| Recurrent thrombosis, % | 33.2 | .... | |
| Primary thrombosis, *% | 25.0 | .... | |
| Factor V Arg506Gln, % | 18.6 | 4.2 | <0.0001 |
| Factor II G20210A Mutation % | 11.9 | 4.5 | 0.0003 |

| | | | |
|---|---|---|---|
| *Excluding acquired risk factors: pregnancy, cancer, surgery, and immobilization OC= oral anticontraceptive HRT= hormonal replacement treatment | | | |

The factor V Arg506Gln mutation was detected in 18.6% of cases and 4.2% of controls (P<0.0001 ), and the prothrombin G20210A mutation in 11.9% of cases and 4.3% of controls (P= 0.0003). These frequencies are similar to those observed in other European populations.

To identify subjects bearing an A3 allele, the inventors developed a rapid screening method for this haplotype (for details see Figure 5). Using this method, the inventors identified 89 patients (26.3%) carrying at least one A3 allele (4 were homozygous and 85 "heterozygous"); 60 controls (17.7%) carried an A3 allele (2 were homozygous and 58 "heterozygous") (P=0.009) (Table 2).

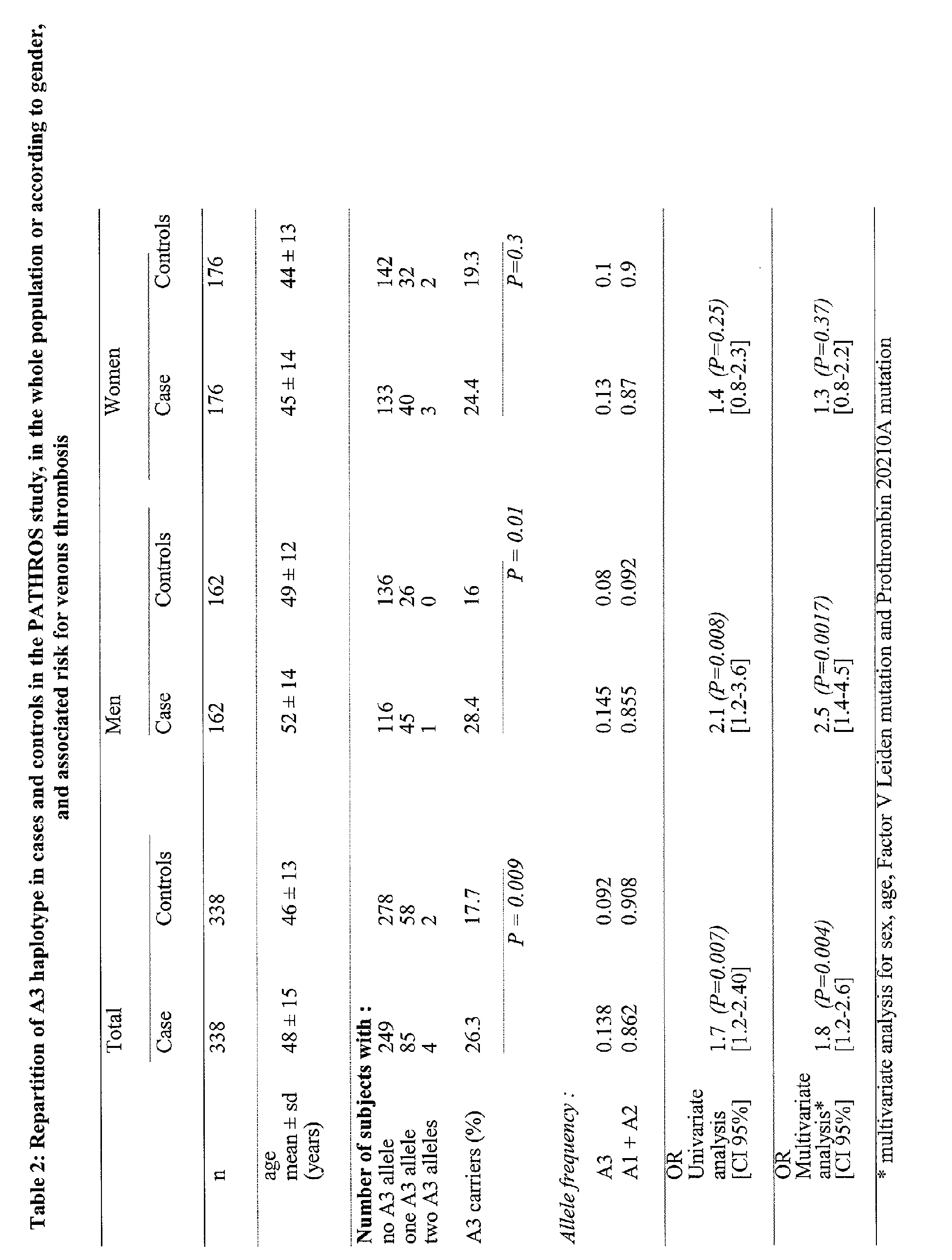

The allelic frequency of the A3 allele was 0.092 in the control subjects and 0.138 in the patients.

Surprisingly, the A3 haplotype distribution was gender-related, prompting the inventors to analyze men and women separately (Table 2). The A3 haplotype distribution remained significantly different (P=0.011) between male cases (28.4% were A3 carriers; allelic frequency 0.145) and male controls (16% were A3 carriers; allelic frequency 0.08). In contrast, there was no significant difference (P=0.3) between female cases (24.4% of A3 carriers; allelic frequency 0.13) and female controls (19.3% of A3 carriers; allelic frequency 0.1).

This latter finding led the inventors to examine whether the A3 haplotype was also associated with higher plasma sEPCR levels in women. They therefore assayed sEPCR in plasma from the 176 female controls (figure 6). Like in the 100 healthy male volunteers population, the healthy female controls from the PATHROS study who carried the A3 haplotype had elevated sEPCR levels. In addition, sEPCR levels correlated with the number of A3 alleles: values were 77.5 ± 20.67 ng/ml (mean±1 SD) in women with no A3 alleles (n= 142), 211.1 ± 48.9 ng/ml in women with one A3 allele (n=32), and 483.2 ± 47.4 ng/ml in the two women who were homozygous for the A3 haplotype (P<0.0001). Thus, as in healthy men, the A3 haplotype is associated with elevated sEPCR levels in healthy women.

However, the frequency of hormonal treatment (oral contraception or replacement therapy) was higher in the female controls than in the female cases. This hinders the interpretation of the above results in the absence of data on the possible interaction of the A3 allele with hormonal treatment.

The inventors expect that the A3 allele is associated with a thrombosis risk in women without hormonal treatment, as it is in men.

### REFERENCES

- Akar N. Endothelial Cell Protein C Receptor (EPCR) Gene Exon III, 23 Bp Insertion Mutation in the Turkish Pediatric Thrombotic Patients. Thromb Haemost. 2002;88:1068-1069.
- Alhenc-Gelas M, Arnaud E, Nicaud V, *et al.* Venous thromboembolic disease and the prothrombin, methylene tetrahydrofolate reductase and factor V genes. Thromb Haemost. 1999;81:506-510.
- Antonarakis et al. (1989), N. Engl. J. Med. 320:153-163 Diagnosis of genetic disorders at the DNA level
- Arnaud E, Nicaud V, Poirier O, et al. Protective effect of a thrombin receptor (Protease-activated receptor 1) gene polymorphism toward venous thromboembolism. Arterioscler Thromb Vasc Biol. 2000;20:585-595.
- Biguzzi E, Gu JM, Merati G, Esmon N, Esmon CT. Point mutations in the endothelial protein C receptor (EPCR) promoter. Thromb Haemost 2002;87:1085-1086.
- Biguzzi E, Merati G, Liaw PC, *et al.* A 23bp insertion in the endothelial protein C receptor (EPCR) gene impairs EPCR function. Thromb Haemost. 2001;86:945-948.
- Cooper et al. (1991) Diagnosis of genetic disease using recombinant DNA, 3rd edition, Hum. Genet, 87:519-560
- Dahlbäck B. The protein C anticoagulant system: inherited defects as basis for venous thrombosis. Thromb Res. 1995;77:1-43.
- Dupont A, Fontana P, Bachelot-Loza C, et *al.* An intronic polymorphism in the PAR-1 gene is associated with platelet receptor density and the response to SFLLRN. Blood. 2003;101:1833-1840.
- Emmerich et al. Combined effect of factor V Leiden and prothrombin 20210A on the risk of venous thromboembolism - pooled analysis of 8 case-control studies including 2310 cases and 3204 controls Thromb. Haemost. 2001 86:809-816
- Espana F, Medina P, Mira Y, *et* al. A new polymorphism in the 3'UTR region of the endothelial protein C receptor associated with increased levels of circulating activated protein C and decreased risk of venous thrombosis. [abstract] Thromb Haemost, 2001; suppl:OC885.
- Espana F, Vaya A, Mira Y, *et al.* Low level of circulating activated protein C is a risk factor for venous thromboembolism. Thromb Haemost. 2001;86:1368-1373.
- Fontana P, Dupont A, Gandrille S, *et al.* ADP-induced platelet aggregation is associated with P2Y12 gene sequence variations in healthy subjects. Circulation. 2003, 108:989-995
- Fukudome K, Esmon CT. Identification, cloning, and regulation of a novel endothelial cell protein C/activated protein C receptor. J Biol Chem 1994 ;269 :26486-26491.
- Fukudome K, Kurosawa S, Stearns-Kurosawa DJ, He X, Rezaie AR, Esmon CT. The endothelial cell protein C receptor. Cell surface expression and direct ligand binding by the soluble receptor. J Biol Chem. 1996;271:17491-17498.
- Fukudome K, Ye X, Tsuneyoshi N, *et al.* Activation mechanism of anticoagulant protein C in large blood vessels involving the endothelial cell protein C receptor. J Exp Med. 1998;187:1029-1035.
- Galligan L, Livingstone W, Mynett-Johnston L, Smith OP. Prevalence of the 23 bp endothelial protein C receptor (EPCR) gene insertion in the Irish population. Thromb haemost 2002;87:773-774.
- Galligan L, Powell C, Livingstone W, Mynett-Johnston L, Smith OP. The G7763C endothelial protein C receptor (EPCR) gene mutation : prevalence and association with DVT in the irish population. Thromb Haemost. 2002;88:163-165.
- Grompe M. The rapid detection of unknown mutations in nucleic acids (1993) Nat. Genet. 5(2):111-7
- Gu JM, Katsura Y, Ferrell GL, Grammas P, Esmon CT. Endotoxin and thrombin elevate rodent endothelial cell protein C receptor mRNA levels and increase receptor shedding in vivo. Blood. 2000;95:1687-1693.
- Hayashi T, Nakamura H, Okada A, *et al.* Organization and chromosomal localization of the human endothelial protein C receptor gene. Gene. 1999;238:367-373.
- Kuklin et al. Detection of single-nucleotide polymorphisms with the WAVE DNA fragment analysis system Genet. Test (1997-98), 1(3):201-6
- Kurosawa S, Stearns-Kurosawa DJ, Carson CW, D'Angelo A, Della Valle P, Esmon CT. Plasma levels of endothelial cell protein C receptor are elevated in patients with sepsis and systemic lupus erythematosus: lack of correlation with thrombomodulin suggests involvement of different pathological processes [letter]. Blood. 1998;91:725-727.
- Kurosawa S, Stearns-Kurosawa DJ, Hidari N, Esmon CT. Identification of functional endothelial protein C receptor in human plasma. : J Clin Invest. 1997;100:411-418.
- Laszik Z, Mitro A, Taylor FB Jr, Ferrell G, Esmon CT. Human protein C receptor is present primarily on endothelium of large blood vessels: implications for the control of the protein C pathway. Circulation. 1997;96:3633-3640.
- Liaw PC, Mather T, Oganesyan N, Ferrell GL, Esmon CT. Identification of the protein C/activated protein C binding sites on the endothelial cell protein C receptor. Implications for a novel mode of ligand recognition by a major histocompatibility complex class 1-type receptor. J Biol Chem. 2001;276:8364-8370.
- Liaw PC, Neuenschwander PF, Smirnov MD, Esmon CT. Mechanisms by which soluble endothelial cell protein C receptor modulates protein C and activated protein C function. J Biol Chem. 2000;275:5447-5452.
- Lin LI. A concordance correlation coefficient to evaluate reproducibility. *Biometrics.* 1989;45:255-268.
- Medina et al The 4678G/C polymorphism in the endothelial protein C receptor (EPCR) gene is associated wit levels of soluble plasma EPCR J. Thromb. Haemost. 2003, suppl 1, P0026
- Mercier et al. Direct PCR from whole blood, without DNA extraction Acids Research 1990 18(19) :5908
- Miller SA, Dykes DD, Polesky HF. A simple salting out procedure for extracting DNA from human nucleated cells. Biochem J. 1998 ; 330 : 1469-1474.
- Navarro S, Medina P, Vaya A, Estelles A, Villa P, Mira Y, Ferrando F, Aznar J, Bertina RM, Espana F. The 4678 G/C polymorphism in the endothelial protein C receptor (EPCR) gene reduces the risk of venous thromboembolism in carriers of the factor V Leiden mutation. J Thromb Haemost 2003, suppl 1 OC 057.
- O'Donnell-Maloney et al. Microfabrication and array technologies for DNA sequencing and diagnosis. Genet. Anal. 1996 ; 13:151-7
- Oganesyan et al. The crystal structure of the endothelial protein C receptor and a bound phospolipid J. Biol. Chem. 2002 277:24851-24854
- Poort SR, Vos HL, Rosendaal FR, Bertina RM. The endothelial protein C receptor (EPCR) 23 bp insert mutation and the risk of venous thrombosis. Thromb Haemost 2002;88:160-162.
- Rance JB, Follows GA, Cockerill PN, Bonifer C, Lane DA, Simmonds RE. Regulation of the human endothelial cell protein C receptor gene promoter by multiple Sp1 binding sites. Blood. 2003;101:4393-4401.
- Regan LM, Stearns-Kurosawa DJ, Kurosawa S, Mollica J, Fukudome K, Esmon CT. The endothelial cell protein C receptor. Inhibition of activated protein C anticoagulant function without modulation of reaction with proteinase inhibitors. J Biol Chem. 1996;271:17499-17503.
- Sambrook et al. (1989) Molecular Cloning: A Laboratory Manual
- Simmonds RE, Lane DA. Structural and functional implications of the intron/exon organization of the human endothelial cell protein C/activated protein C receptor (EPCR) gene: comparison with the structure of CD1/major histocompatibility complex alpha1 and alpha2 domains. Blood. 1999;94:632-641.
- Stearns-Kurosawa DJ, Burgin C, Parker D, Comp P, Kurosawa S. Bimodal distribution of soluble endothelial protein C receptor levels in healthy populations [letter]. Blood 2003 ;4: 855-856.
- Stearns-Kurosawa DJ, Kurosawa S, Mollica JS, Ferrell GL, Esmon CT. The endothelial cell protein C receptor augments protein C activation by the thrombin-thrombomodulin complex. Proc Natl Acad Sci U S A. 1996;93:10212-10216.
- Stearns-Kurosawa DJ, Swindle K, D'Angelo A, et al. Plasma levels of endothelial protein C receptor respond to anticoagulant treatment. Blood. 2002;99:526-530.
- Taylor FB Jr, Peer GT, Lockhart MS, Ferrell G, Esmon CT. Endothelial cell protein C receptor plays an important role in protein C activation in vivo. Blood. 2001;97:1685-1688.
- Taylor FB Jr, Stearns-Kurosawa DJ, Kurosawa S, *et al*. The endothelial cell protein C receptor aids in host defense against Escherichia coli sepsis. Blood. 2000;95:1680-1686.
- Villoutreix BO, Blom AM, Dahlback B. Structural prediction and analysis of endothelial cell protein C/activated protein C receptor. Protein Eng. 1999;12:833-840.
- von Depka M, Czwalinna A, Eisert R, et *al.* Prevalence of a 23bp insertion in exon 3 of the endothelial cell protein C receptor gene in venous thrombophilia. Thromb Haemost. 2001;86:1360-1362.
- Xu J, Qu D, Esmon NL, Esmon CT. Metalloproteolytic release of endothelial cell protein C receptor. J Biol Chem. 2000;275:6038-6044.
- Ye X, Fukudome K, Tsuneyoshi N, *et al.* The endothelial cell protein C receptor (EPCR) functions as a primary receptor for protein C activation on endothelial cells in arteries, veins, and capillaries. Biochem Biophys Res Commun. 1999;259:671-677.

## Claims

1. An *in vitro* method for determining the risk of developing thrombosis in a subject, which method comprises identifying polymorphisms of EPCR gene on at least one of positions 1651, 3610, 4216, and 6936 (SEQ ID No:1 ), wherein the presence of G at position 1651, C at position 3610, A at position 4216, or G at position 6936 is indicative of a higher risk to develop thrombosis in comparison with a control subject that does not show the same polymorphisms.

2. An *in vitro* method according to claim 1, which method comprises identifying polymorphisms of EPCR gene at positions 1651, 3610, 4216, 6936, 3787, 3877, 4868, 5233, 5760, 6333, 7014, 7968, 7999 of SEQ ID No: 1, wherein the simultaneous presence of :
- G at position 1651
- C at position 3610
- A at position 4216
- G at position 6936
- C at position 3787
- G at position 3877
- T at position 4868
- G at position 5233
- T at position 5760
- T at position 6333
- G at position 7014
- A at position 7968
- G at position 7999
are designated A3 haplotype and, when present on at least one allele, are indicative of a higher risk to develop thrombosis in comparison with a control subject without any A3 allele.

3. The method according to claim 1 or 2, wherein said thrombosis is a venous thrombosis.

4. The method according to any of claims 1 to 3, wherein the analysis is undertaken on genomic DNA that is extracted from a biological sample of the subject.

5. The method according to any of claims 1 to 4, wherein the analysis comprises a step of amplification of the genomic DNA.

6. The method according to any of claims 1 to 5, wherein the polymorphisms of the EPCR gene are identified by sequencing.

7. The method according to any of claims 1 to 6, wherein at least one of the polymorphisms of the EPCR gene is identified by RFLP analysis.

8. The method according to claim 7, comprising identifying the polymorphism of EPCR gene on position 6936, by creating a restriction site for endonuclease *Pstl* by amplification of the EPCR gene with mutagenic primers, when the amplified fragment contains an A at position 6936, so that when the amplified fragment containing a G, it remains undigested.

9. An isolated nucleic acid encoding the EPCR receptor, that comprises SEQ ID No:2.

10. A kit suitable for the methods according to any of claims 1 to 6, which kit comprises a pair of nucleotide primers specific for amplifying all or part of the EPCR gene comprising at least one of positions 1651, 3610, 4216 of SEQ ID No:1.
